# EUROPEAN PATENT SPECIFICATION

(11) **EP 1 232 730 B1**
(45) Date of publication and mention of the grant of the patent: **04.05.2005**
(21) Application number: 02011242.1
(22) Date of filing: 08.01.1998
(51) Int. Cl.: A61B 19/00, A61B 5/06, A61F 13/44

(54) **Surgical implement detector utilizing a smart marker**
Suchgerät für chirurgisches Instrument versehen mit intelligenter Markierung
Détecteur d'instrument chirurgical utilisant un marqueur intelligent

(30) Priority: 08.01.1997 US 780697
(43) Date of publication of application: 21.08.2002
(62) Divisional of application: 98903376.6
(73) Proprietor: FABIAN, Carl E., Miami Shores, FL 33138 (US); ANDERSON, Philip M., Madison, NJ 07940 (US)
(72) Inventor: FABIAN, Carl E., Miami Shores, FL 33138 (US); ANDERSON, Philip M., Madison, NJ 07940 (US)
(74) Representative: Price, Nigel John King

(56) References cited:
- WO-A-94/17767
- US-A- 5 057 095

## Description

The present invention relates to a method for detecting a marked surgical implement such as a sponge, clamp, or catheter within a surgical wound in human or animal tissue irrespective of its position or orientation therewithin.

### 2. Description of the Prior Art

During the course of a surgical operation it is frequently necessary for articles, such as surgical sponges, gauzes, instruments, needles, and the like, to be placed into a wound cavity. Notwithstanding rigorous precautions attendant surgical procedures, such items are sometimes inadvertently lost during surgery and remain within the wound. When this happens the patient can encounter serious consequences, including pain, infection, intestinal obstruction, and even death. The problem of retained surgical implements has existed since the earliest days of surgery. Procedures conventionally employed to prevent post-surgical implement retention include a manual search of the wound cavity by the surgeon prior to closure and a careful accounting for all materials inserted and removed from the wound. The accounting function is customarily carried out by the operating room staff, usually the circulating nurse. Despite these precautionary measures the accidental retention of surgical implements continues to occur with disturbing regularity, even in prestigious institutions, and is regarded by surgeons as a major unsolved problem.

At present, manual search and physical count remain the primary methods used to prevent retention of surgical implements. Most surgical instruments are composed of metal, and are easily detectable by x-ray. Sponges are usually marked with radiopaque markers to make them additionally visible by x-ray. But x-rays are not routinely carried out upon completion of the operation because of several disadvantages, including inconvenience, expense, loss of operative time, and radiation exposure. Performing an x-ray after the patient has left the operating room suffers from some of the same disadvantages. Moreover, even when postoperative x-rays are taken, retained surgical items are frequently overlooked, and even if detected at that time, require a second operation to effect their removal.

To overcome the difficulty of detecting retained surgical implements, it has been suggested that the implements be provided with a radioactive tracer. This technique, disclosed by U. S. Patent 2,740,405 to Riordan, is subject to obvious hazards associated with use, storage and disposal of radioactive materials.

It has also been proposed that surgical sponges be marked with a flexible plastic impregnated with either paramagnetic or ferromagnetic materials in the form of powders. Detection of these marked sponges is accomplished by a metal detector. This method, taught by U. S. Patent 3,422,816 to Robinson et al., provides very small signals difficult to detect over the width of a patient's body. In addition, the Robinson et al. technique provides no discrimination against other metal objects, such as staples which, though present within the surgical wound, are appointed for retention therewithin.

Yet another proposal, advanced by U. S. Patent 3,587,583 to Greenberg, involves use of surgical sponges marked with magnetized particles whose presence is detectable with magnetodiodes. In practice, however, the magnetic field generated by these particles is too small to be readily detected by the diodes.

U. S. Patent 4,114,601 to Abels discloses the use of a small transponder fixed to a surgical sponge or instrument. This transponder exhibits gyromagnetic resonance at preselected frequencies. Detection is accomplished by nonlinear mixing of two frequencies impinging upon the transponder. The gyromagnetic resonance effect disclosed by Abels is a high frequency phenomenon, existing at frequencies of the order of about 5 gigahertz (5,000,000,000 cycles/sec). These frequencies, known as microwaves, are absorbed readily by animal tissue and are, in fact, used in microwave ovens for cooking. In use of the Abels type transponder, the energy developed goes primarily into heating tissue, rather than exciting the transponder into gyromagnetic resonance.

British Patent No. 2,242,551 discloses a surgical implement having a bar code attached thereto. The bar code is optically read and oftentimes becomes obscured by blood and tissue fragments within the wound cavity. Hence its use is generally impractical for surgical procedures.

U. S. Patent 5,057,095 to Fabian and Anderson discloses the use of a resonant transponder fixed to a surgical sponge or instrument, and U. S. Patent 5,190,059 to Fabian and Anderson discloses a powered marker, while U. S. Patent 5,188,126 to Fabian and Anderson discloses a capacitive marker. These patents do not provide for storing a code in a marker.

### Summary of the Invention

The present invention provides a method and apparatus for accurately and reliably accounting for surgical implements used during a surgical operation.

According to the invention, there is provided an apparatus adapted to account for a surgical implement appointed for disposition in human or animal tissue. Generally stated, the apparatus comprises a marker adapted to be secured to the surgical implement. The marker has a preprogrammed code. A microcontroller means adapted for electrical connection to the marker reads and changes the preprogrammed code into a reprogrammed code. An indicating means indicates the reprogrammed code. The apparatus further comprises memory means for holding the preprogrammed code. A multiplexer means in communication with the memory means decodes the preprogrammed code and encodes the reprogrammed code. The marker has a connection point on an external surface and a ground connection point on a different portion of the external surface. The connection point cooperates with the ground connection point to establish the electrical connection.

Further provided is a method of accounting for a surgical implement appointed for disposition in human or animal tissue. The method is as defined in claim 3.

Advantageously, the method and apparatus of the invention detect retained surgical implements with far greater accuracy and efficiency than methods and means involving a physical count of implements that enter and exit the wound. Coding of the marker uniquely differentiates it from other markers, assuring positive identification and specification of the implement to which it is attached. The apparatus is inexpensive to construct, more efficient and reliable than postoperative X-rays and avoids risk to the environment and personnel posed by radioactive tracers. The signal is more easily distinguished than signals generated by magnetic detection systems, and is generated without the heating of tissue caused by microwave detection systems.

### Brief Description of the Drawings

The invention will be more fully understood and further advantages will become apparent when reference is made to the following detailed description of the preferred embodiment of the invention and the accompanying drawings in which:
Fig. 1 is a block diagram of a surgical implement detector reader and marker incorporating the elements of the present invention;
Fig. 2 is a schematic diagram depicting an alternate antenna configuration for the detector of Fig. 1;
Fig. 3 is a circuit diagram of a marker suited for use in the detector of Fig. 1;
Fig. 4 is a block diagram showing an alternate embodiment of a surgical implement detector reader; and
Fig. 5 is an alternate embodiment of the invention shown in Fig. 1, where direct contact between marker and reader is established.

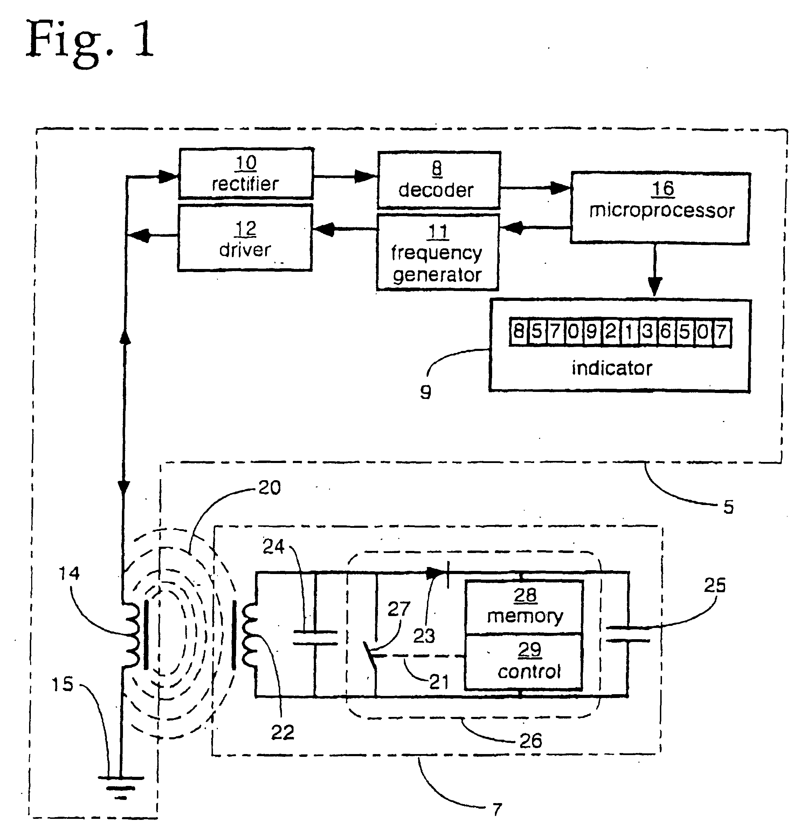

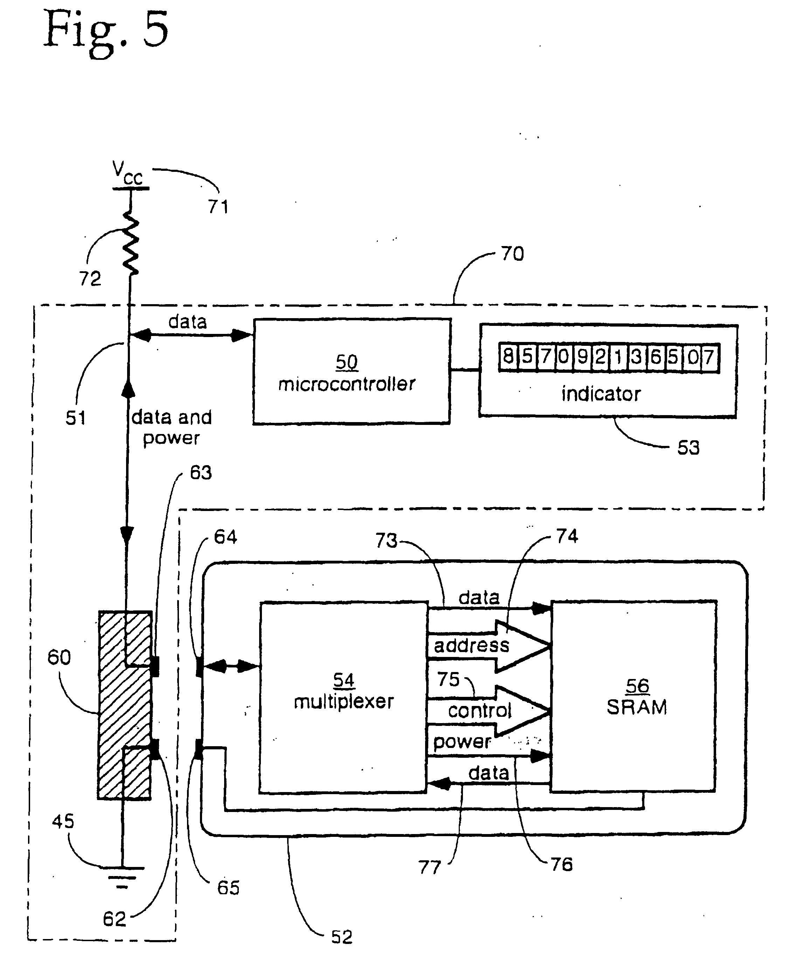

Figures 1-4 do not illustrate the present invention but are useful for understanding of figure 5.

### Description of the Preferred Embodiments

Referring to the drawings, there is shown in Fig. 1 an apparatus for detecting a surgical implement in human or animal tissue Frequency generator 11, driver 12, and reader antenna 14 provide means for generating magnetic field 20 at a predetermined frequency. Rectifier 10, provide a means for receiving the predetermined code of marker 7, and decoder 8 provides a means for decoding the predetermined code. Microprocessor 16 provides the means for verifying the predetermined code. Indicator 9 provides a means for indicating reception of the predetermined code. Alternatively, verification of the predetermined code can be performed manually.

In Fig. 1, reader 5 comprises microprocessor 16, frequency generator 11, driver 12, rectifier 10, decoder 8, indicator 9, and reader antenna 14. The reader is shown by the dashed box labeled 5 in Fig. 1. Microprocessor 16 directs frequency generator 11 to provide a predetermined frequency to driver 12. Preferably, the predetermined frequency is between 50 kHz and 300 kHz. Driver 12 in turn supplies reader antenna 14 with a current of the predetermined frequency, thus in turn, generating magnetic field 20 having the predetermined frequency. Preferably, reader antenna 14 is comprised of a coil with a ferrite core.

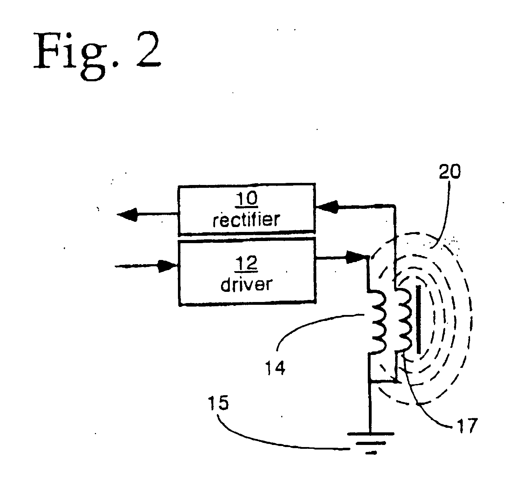

Marker 7 comprises marker antenna 22, tuning capacitor 24, diode 23, capacitor 25, memory 28, control 29, and switch 27. The marker is indicated by the dashed box labeled 7 in Fig. 1. Preferably, memory 28 is an EEPROM provided with a predetermined code. Marker antenna 22 is comprised of a coil and ferrite core having dimensions preferably near 6 by 30 mm. Tuning capacitor 24 forms, with marker antenna 22, a tuned tank circuit having a resonant frequency equal to the predetermined frequency of magnetic field 20. When marker 7 is sufficiently close to reader antenna 14, magnetic field 20 will generate a voltage in marker antenna 22 that is rectified by diode 23 causing capacitor 25 to charge. When capacitor 25 reaches a predetermined voltage, memory 28, and control 29 become operational. The predetermined voltage is typically between 3 and 5 volts, depending on the choice of marker components. Control 29, via connection 21, causes switch 27 to open and close in sequence according to the predetermined code of memory 28. The opening and closing of switch 27 causes marker antenna 22 to vary impedance. This varying impedance disturbs magnetic field 20, which in turn disturbs the voltage from driver 12 in reader antenna 14. The varying voltage is rectified by rectifier 10. Decoder 8 filters and demodulates the voltage to reconstruct the predetermined code of memory 28. Microprocessor 16 determines validity of the code by comparing the code with a programmed internal code list. If a match is found, indicator 9 is activated. Preferably, decoder 8 and frequency generator 11 are combined as an integrated circuit of the type m3394b. The m3394b requires a quartz crystal and a maximum of 2 RC support circuits. Alternatively as shown in Fig. 2, reader antenna 14 comprises a second coil 17 connected to rectifier 10 for sensing the disturbance of magnetic field 20. Circuit ground is indicated by 15 in Fig. 1.

Preferably as shown in Fig. 1, diode 23, memory 28, control 29, and switch 27 are combined together into a single integrated circuit 26 in marker 7. The integrated circuit is indicated by the dashed box labeled 26 in Fig. 1.

For marker antenna 22 to be effective, the predetermined frequency of magnetic field 20 should be below 3 megahertz and above 5 kilohertz. Preferably, the predetermined frequency of magnetic field 20 ranges from about 50 to 300 kilohertz. It is even more preferable that the predetermined frequency of magnetic field 20 be near or at 125 kilohertz.

The method for detecting a surgical implement in human or animal tissue comprises the steps of: generating a magnetic field having a predetermined frequency; attaching a marker to a surgical implement appointed for disposition within the tissue, the marker being powered by the magnetic field and being operative in its presence to transmit a predetermined code; receiving the predetermined code; decoding the predetermined code; verifying the predetermined code; and indicating receipt of the predetermined code.

In practice, a marker is attached to a surgical implement and the reader is used to read and record the marker's predetermined code to a inventory list in the microprocessor. On removal of a marker from the surgical wound, the reader is again used to read and record the predetermined code, which is matched with the inventory list. Unmatched codes indicate unaccounted for implements. The user is alerted by the mismatch, and a search of the wound with the reader is made to locate any missing implements. Prior to closure of the surgical wound, the reader is placed near the wound to perform a fail-safe search for implements.

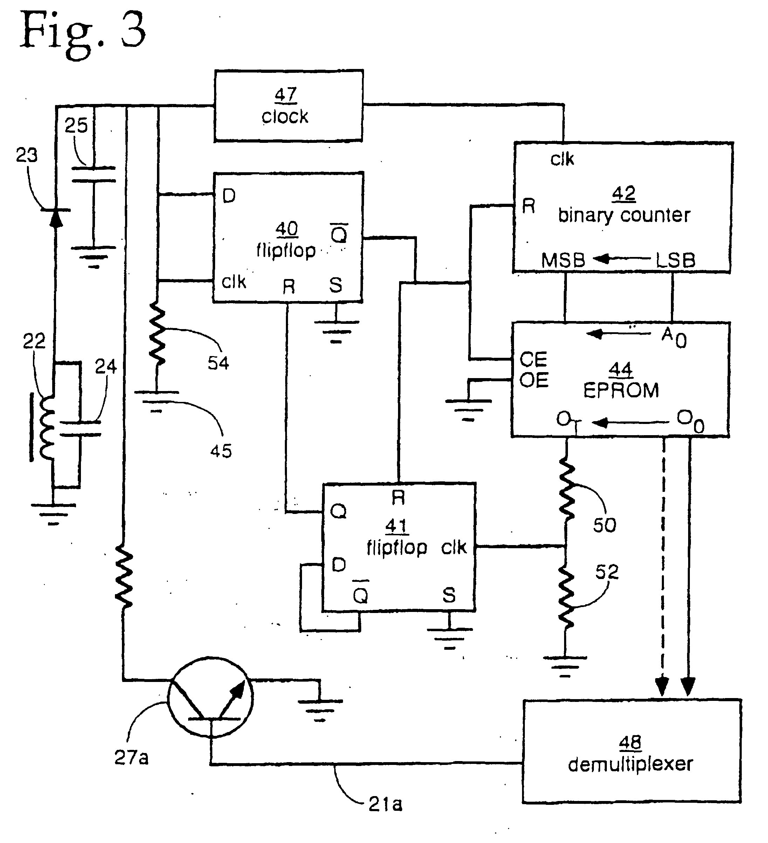

. Figure 3 shows one possible circuit for marker 7. A voltage induced in marker antenna 22 and tuning capacitor 24, and rectified by diode 23 charges capacitor 25. Capacitor 25 is now the source of power for the other elements of marker 7. Clock 47 provides circuit timing. Flip-flop 40 causing initialization of binary counter 42 and EEPROM 44. With each clock pulse from clock 47, binary counter 42 counts up from zero to one plus the number of digits of the predetermined code. With each count, EEPROM 44 outputs the corresponding digit of the predetermined code to demultiplexer 48. The last digit in memory of EEPROM 44 provides an end-of-sequence pulse to flip-flop 41, which in turn provides a reset pulse to binary counter 42 and EEPROM 44. Demultiplexer 48 sends each digit of the predetermined code along control line 21a to switch 27a. In this manner, marker antenna 22 is either shorted or left unaffected depending on the digit of the predetermined code considered. Resistors 54, 50, and 52 provide reference voltages with respect to ground 45.

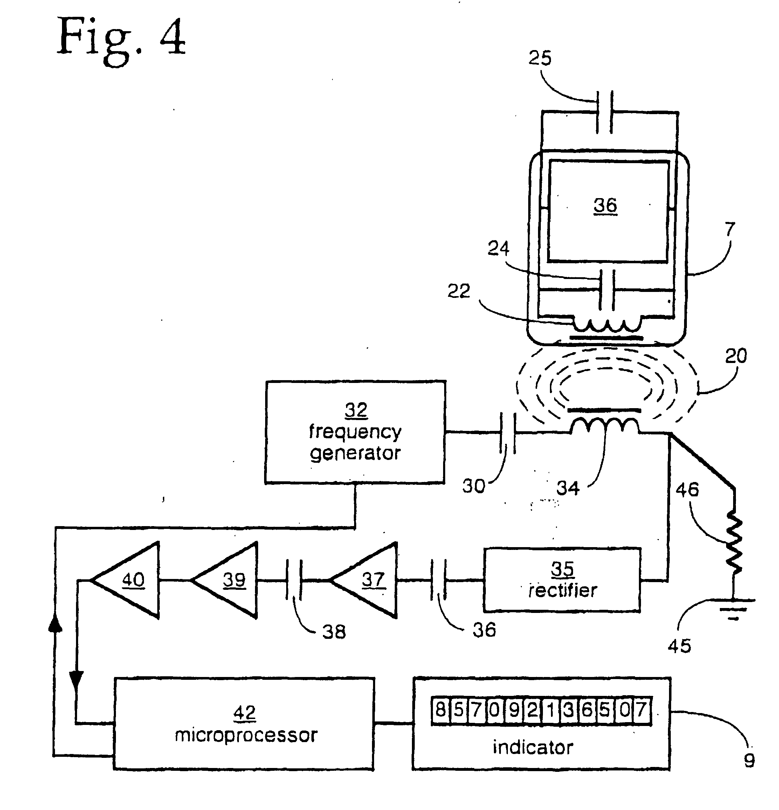

Fig. 4 shows an alternate construction of reader 5. Frequency generator 32 is coupled to antenna 34 through capacitor 30 creating magnetic field 20. Marker 7 disturbs magnetic field 20 as described above. The disturbed voltage is sensed and rectified by rectifier 35. Tuned amplifiers 37 and 39 along with coupling capacitors 36 and 38 filter and demodulate the sensed disturbance voltage. Comparator 40 reproduces the predetermined code transmitted from marker 7 and inputs the code to microprocessor 42. Microprocessor 42 verifies the code with comparison to a programmed internal code list and indicates the comparison product on indicator 9. Resistor 46 provides voltage difference with ground 45.

Fig. 5 shows an embodiment of the invention, in which the code contained in marker 52 is read or changed by electrically connecting reader/programmer 70 to marker 52. Reader/programmer, indicated by dashed box labeled 70, comprises microcontroller 50, indicator 53, data line 51, and connector 60. A means for electrically connecting marker 52 and microcontroller 50 is provided by connector 60. Connector 60 comprises data/power connector 63 and ground connector 62 which is connected to ground 45. Marker 52 comprises multiplexer 54, a memory means 56, and connection point 64. and ground connection point 65. While memory means 56 can be implemented using a different technology, i.e. EEPROM, the memory means 56 preferably comprises static random access memory or SRAM. In order to read or change the code contained within marker 52, data/power connector 63 must connect directly to connection point 64 of marker 52, and ground connector 62 must connect directly to ground connection point 65 of marker 52. Thus, microcontroller 50 is electrically connected to marker 52 via line 51 and connector 60. Voltage source 71 and drop-down resistor 72 provide power for the circuit.

When reading, microcontroller 50 sends a read query to marker 52. The read query comprises a read control command and an address to multiplexer 54, which in turn channels the read query through line 75, and the address through line 74 to SRAM 56. SRAM 56 in turn provides the data residing at the queried address through line 77 to multiplexer 54 and then in turn to microcontroller 50. Microcontroller 50 repeats the read query until all of the code contained within marker 52 is read. In this manner, multiplexer 54 decodes the preprogrammed code of SRAM 56. Microcontroller 50 also verifies the read code by comparing with an internal program list. Power for SRAM 56 is provided through line 76.

When changing a code, microcontroller 50 sends a write query to marker 52. The write query comprises a write control command, an address, and a code digit to multiplexer 54, which in turn channels the write query through line 75, the address through line 74, and the code digit through line 73 to SRAM 56. Microcontroller 50 repeats the write query until all of the code is written to marker 52. In this manner, multiplexer 54 encodes a reprogrammed code to SRAM 56.

In practice, a marker is attached to a surgical implement. Before use in an operation, operating room personnel read the code of the marker by connecting it to the reader/programmer and reading and recording the marker code. When removing the implement, the marker code is again read and recorded. In this way an accounting can be efficiently kept of the implements used during the surgery and missing implements are readily identified.

The invention additionally provides a method of accounting for a surgical implement appointed for disposition in human or animal tissue, comprising the steps of: electrically connecting a marker to a reader, the marker having a preprogrammed code and being secured to a surgical implement appointed for disposition within the tissue; reading the preprogrammed code; decoding the preprogrammed code; verifying the preprogrammed code; and indicating receipt of the preprogrammed code.

The surgical implement detector disclosed herein can, of course, be modified in numerous ways without departing from the scope of the invention. For example, an optical scanner and bar code can be added to provide back-up redundancy and/or permit alternative information gathering capacity before or after the surgical procedure, or at locations remote from the wound cavity. Such modifications are intended to fall within the scope of the invention as defined by the subjoined claims.

Having thus described the invention in rather full detail, it will be understood that such detail need not be strictly adhered to but that various changes and modifications may suggest themselves to one skilled in the art, all falling within the scope of the invention as defined by the subjoining claims.

## Claims

1. Apparatus adapted to account for a surgical implement appointed for disposition in human or animal tissue, comprising:
(a) a marker (52) secured to said surgical implement, said marker having a preprogrammed code;
(b) microcontroller means (50,60) adapted for electrical connection to said marker for reading and changing said preprogrammed code into a reprogrammed code; and
(c) indicating means (53) for indicating said reprogrammed code.

2. Apparatus as recited by claim 1, wherein said marker further comprises:
(d) memory means (56) for holding said preprogrammed code;
(e) multiplexer means in communication with said memory means for decoding said preprogrammed code and encoding said reprogrammed code; and
(f) a connection point on an external surface of said marker and a ground connection point on a different portion of said external surface, said connection point cooperating with said ground connection point to establish said electrical connection.

3. A method of accounting for a surgical implement appointed for disposition in human or animal tissue, comprising the steps of:
(a) electrically connecting a marker as defined in claim 1 to a microcontroller means as defined in claim 1, said marker having a preprogrammed code and being secured to a surgical implement appointed for disposition within said tissue;
(b) reading said preprogrammed code;
(c) decoding said preprogrammed code;
(d) verifying said preprogrammed code; and
(e) indicating receipt of said preprogrammed code.

## Patentansprüche

1. Vorrichtung, welche zum Nachweis eines zur Verwendung in menschlichem oder tierischem Gewebe bestimmten chirurgischen Instruments ausgelegt ist, welche umfasst:
(a) eine an dem chirurgischen Instrument befestigte Markierungseinrichtung (52), welche einen vorprogrammierten Code aufweist;
(b) für den elektrischen Anschluss an der Markierungseinrichtung ausgelegte Mikrocontrollermittel (50, 60) für das Lesen und Ändern des vorprogrammierten Codes in einen umprogrammierten Code; und
(c) Anzeigemittel (53) für das Anzeigen des umprogrammierten Codes.

2. Vorrichtung nach Anspruch 1, **dadurch gekennzeichnet, dass** die Markierungseinrichtung weiterhin umfasst:
(d) Speichermittel (56) für das Festhalten des vorprogrammierten Codes;
(e) Multiplexermittel in Verbindung mit dem Speichermittel für das Decodieren des vorprogrammierten Codes und des Codieren des umprogrammierten Codes; und
(f) einen Anschlusspunkt an einer Außenfläche der Markierungseinrichtung und einen Erdungspunkt an einem anderen Teil der Außenfläche, wobei der Anschlusspunkt mit dem Erdungspunkt zusammen das Herstellen des elektrischen Anschlusses bewirkt.

3. Verfahren für das Nachweisen eines zur Verwendung in menschlichem oder tierischem Gewebe bestimmten chirurgischen Instruments, welches die folgenden Schritte umfasst:
(a) elektrisches Anschließen einer Markierungseinrichtung nach Anspruch 1 an einem Mikrocontrollermittel nach Anspruch 1, wobei die Markierungseinrichtung einen vorprogrammierten Code aufweist und an einem zur Verwendung in dem Gewebe bestimmten chirurgischen Instrument befestigt ist;
(b) Lesen des vorprogrammierten Codes;
(c) Decodieren des vorprogrammierten Codes;
(d) Überprüfen des vorprogrammierten Codes auf Richtigkeit; und
(e) Anzeigen des Empfangs des vorprogrammierten Codes.

## Revendications

1. Dispositif adapté pour détecter un instrument chirurgical conçu pour être placé dans un tissu humain ou animal, comprenant :
(a) un marqueur (52) fixé au dit instrument chirurgical, ledit marqueur ayant un code préprogrammé ;
(b) un moyen (50, 60) formant microcontrôleur adapté pour un raccordement électrique audit marqueur pour lire et modifier ledit code préprogrammé en un code reprogrammé ; et
(c) un moyen (53) d'indication pour indiquer ledit code reprogrammé.

2. Dispositif selon la revendication 1, dans lequel ledit marqueur comprend en outre :
(d) un moyen (56) de mémoire pour contenir ledit code préprogrammé ;
(e) un moyen multiplexeur en communication avec ledit moyen de mémoire pour décoder ledit code préprogrammé et coder ledit code reprogrammé ; et
(f) un point de raccordement sur une surface externe dudit marqueur et un point de raccordement à la terre sur une partie différente de ladite surface externe, ledit point de raccordement coopérant avec ledit point de raccordement à la terre pour établir ledit raccordement électrique.

3. Procédé pour détecter un instrument chirurgical conçu pour être placé dans un tissu humain ou animal, comprenant les étapes consistant à :
(a) raccorder électriquement un marqueur tel que défini dans la revendication 1 à un moyen formant microcontrôleur tel que défini dans la revendication 1, ledit marqueur ayant un code préprogrammé et étant fixé à un instrument chirurgical conçu pour être placé dans ledit tissu ;
(b) lire ledit code préprogrammé ;
(c) décoder ledit code préprogrammé ;
(d) vérifier ledit code préprogrammé ; et
(e) indiquer la réception dudit code préprogrammé.
